# FASCICULE DE BREVET EUROPEEN

(11) **EP 2 058 296 B1**
(45) Date de publication et mention de la délivrance du brevet: **05.10.2016**
(21) Numéro de dépôt: 08291042.3
(22) Date de dépôt: 07.11.2008
(51) Int. Cl.: C07C 233/18, A61K 9/20

(54) **Nouvelle forme cristalline VI de l'agomélatine, son procédé de préparation et les compositions pharmaceutiques qui la contiennent**
Neue kristalline Form VI von Agomelatin, ihr Herstellungsverfahren und pharmazeutische Zusammensetzungen, die sie enthalten
New crystalline form VI of agomelatine, method of preparation and pharmaceutical compositions thereof

(30) Priorité: 09.11.2007 FR 0707861
(43) Date de publication de la demande: 13.05.2009
(73) Titulaire: Les Laboratoires Servier, 92284 Suresnes Cedex (FR)
(72) Inventeur: Coquerel, Gérard, 76520 Boos (FR); Linol, Julie, 76100 Rouen (FR); Le Pape, Lionel, 76970 Gremonville (FR); Lecouve, Jean-Pierre, 76600 Le Havre (FR)

(56) Documents cités:
- EP-A- 0 447 285
- EP-A- 1 564 202
- EP-A- 1 752 444
- DEPREUX P ET AL: "SYNTHESIS AND STRUCTURE-ACTIVITY RELATIONSHIPS OF NOVEL NAPHTHALENIC AND BIOISOSTERIC AMIDIC DERIVATIVES AS MELATONIN RECEPTOR LIGANDS" JOURNAL OF MEDICINAL CHEMISTRY, AMERICAN CHEMICAL SOCIETY. WASHINGTON, vol. 37, no. 20, 30 septembre 1994 (1994-09-30), pages 3231-3239, XP002016146 ISSN: 0022-2623
- TINANT B ET AL: "N-Ä2-(7-METHOXY-1-NAPHTHYL)ETHYLÜACETAMID E, A POTENT MELATONIN ANALOG" ACTA CRYSTALLOGRAPHICA SECTION C. CRYSTAL STRUCTURECOMMUNICATIONS, MUNKSGAARD, COPENHAGEN, DK, vol. C50, no. 6, 1 janvier 1994 (1994-01-01), pages 907-910, XP009047983 ISSN: 0108-2701
- THRELFALL ET AL: "Analysis of Organic Polymorphs" ANALYST, LONDON, GB, vol. 120, 1 octobre 1995 (1995-10-01), pages 2435-2460, XP002101807

## Description

La présente invention concerne une nouvelle forme cristalline VI de l'agomélatine ou *N-*[2-(7-méthoxy-1-naphtyl)éthyl]acétamide de formule (I) : 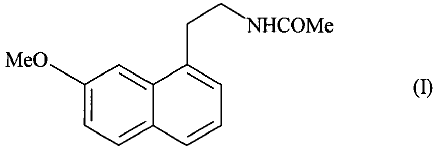 son procédé de préparation ainsi que les compositions pharmaceutiques qui la contiennent. L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confère une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité. L'agomélatine, sa préparation et son utilisation en thérapeutique ont été décrits dans le brevet européen EP 0 447 285.

Des formes cristallines de l'agomélatine sont déjà divulguées dans l'art antérieur, comme par exemple EP1752444.

Compte tenu de l'intérêt pharmaceutique de ce composé, il était primordial de l'obtenir avec une excellente pureté, et notamment sous une forme parfaitement reproductible, présentant des caractéristiques intéressantes de dissolution et de facilité de formulation permettant son stockage prolongé sans conditions particulières de température, de lumière, d'humidité ou de taux d'oxygène.

La demanderesse a présentement mis au point un procédé d'obtention de l'agomélatine sous une forme cristalline bien définie, reproductible et présentant de ce fait des caractéristiques intéressantes de dissolution et de facilité de formulation. Cette nouvelle forme présente de plus une tenue dans le temps tout à fait remarquable permettant un stockage optimal sans précautions particulières, ce qui constitue un avantage primordial dans l'industrie pharmaceutique.

Plus spécifiquement, la présente invention concerne la forme cristalline VI du composé de formule (I), caractérisée par le diagramme de diffraction X sur poudre suivant, mesuré sur un diffractomètre Bruker D5000matic (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°) exp.** | **d (Å) exp.** | **Intensité (%)** |
|---|---|---|
| 5,73 | 15,411 | 11,4 |
| 10,22 | 8,645 | 11,6 |
| 20,10 | 4,413 | 10,2 |
| 23,69 | 3,751 | 59,0 |
| 29,48 | 3,027 | 14,8 |

La forme cristalline VI du composé de formule (I) a également été caractérisée par le diagramme de spectroscopie infrarouge suivant : pics observés à 907,5 cm⁻¹ ; 866,7 cm⁻¹ ; 852,8 cm⁻¹ ; 827,4 cm⁻¹ ; 754,6 cm⁻¹ ; 734,6 cm⁻¹ ; 698,4 cm⁻¹ ; 672,1 cm⁻¹ ; 650,9 cm⁻¹ ; 611,9 cm⁻¹ ; 588,1 cm⁻¹.

L'invention s'étend également au procédé de préparation de la forme cristalline VI du composé de formule (I), caractérisé en ce qu'une solution d'agomélatine dans l'éther isopropylique est portée à ébullition, puis refroidie rapidement à 0°C. Après une filtration sous vide, la forme VI est obtenue pure.
Dans le procédé de cristallisation selon l'invention, on peut utiliser le composé de formule (I) obtenu par n'importe quel procédé.

L'invention s'étend également à un autre procédé de préparation de la forme cristalline VI du composé de formule (I), caractérisé en ce que l'on cristallise l'agomélatine dans un mélange eau/éthanol (50/50 volume/volume) à température ambiante sous une haute pression, pendant 24 heures.
De préférence, dans ce second procédé de cristallisation selon l'invention, l'agomélatine sera recristallisé sous une haute pression de 10 Kbar.
Dans ce second procédé de cristallisation selon l'invention, on peut utiliser le composé de formule (I) obtenu par n'importe quel procédé.

L'obtention de cette forme cristalline a pour avantage de permettre la préparation de formulations pharmaceutiques ayant une composition constante et reproductible, présentant une excellente tenue dans le temps.

L'étude pharmacologique de la forme VI ainsi obtenue a montré une importante activité sur le système nerveux central ainsi que sur la microcirculation qui permet d'établir son utilité dans le traitement du stress, des troubles du sommeil, de l'anxiété, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, la forme VI de l'agomélatine peut être utilisée dans les dysfonctionnements sexuels, qu'elle possède des propriétés d'inhibiteurs de l'ovulation, d'immunomodulateurs et qu'elle est susceptible d'être utilisée dans le traitement des cancers.

La forme cristalline VI de l'agomélatine sera utilisée de préférence dans les traitements de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'invention s'étend aussi aux compositions pharmaceutiques renfermant comme principe actif la forme cristalline VI du composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,1 mg à 1 g par jour en une ou plusieurs prises.

Les exemples ci-dessous illustrent l'invention, mais ne la limitent en aucune façon.

### Exemple 1 : Forme cristalline VI du N-[2-(7-Méthoxy-1-naphtyl)éthyl]acétamide

0,74 g de *N*-[2-(7-Méthoxy-1-naphtyl)éthyl]acétamide et 36,06 g d'éther isopropylique sont introduits dans un tube. Cette suspension est portée à ébullition (à une température de 73°C) pendant 2 heures. Puis un refroidissement rapide à 0°C est effectué. Au bout d'une heure à 0°C, une filtration sous vide sur un verre fritté de porosité 3 est effectuée. Le solide obtenu est caractérisé par son point de fusion et par le diagramme de diffraction X sur poudre suivant, mesuré sur un diffractomètre Bruker D5000matic (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°) exp.** | **d (Å) exp.** | **Intensité (%)** |
|---|---|---|
| 5,73 | 15,411 | 11,4 |
| 10,22 | 8,645 | 11,6 |
| 20,10 | 4,413 | 10,2 |
| 23,69 | 3,751 | 59,0 |
| 29,48 | 3,027 | 14,8 |

### Point de fusion : 94°C

Diagramme de spectroscopie infrarouge: 907,5 cm⁻¹ ; 866,7 cm⁻¹ ; 852,8 cm⁻¹ ; 827,4 cm⁻¹ ; 754,6 cm⁻¹ ; 734,6 cm⁻¹ ; 698,4 cm⁻¹ ; 672,1 cm⁻¹ ; 650,9 cm⁻¹ ; 611,9 cm⁻¹ ; 588,1 cm⁻¹.

### Exemple 2 : Forme cristalline VI du N-[2-(7-Méthoxy-1-naphtyl)éthyl]acétamide

2 g de *N*-[2-(7-Méthoxy-1-naphtyl)éthyl]acétamide sont placés dans 20 ml d'un mélange eau/éthanol (50/50 volume/volume) à 25°C. Cette suspension est filtrée sur un verre fritté de porosité 4. Cette solution saturée en *N*-[2-(7-méthoxy-1-naphtyl)éthyl]acétamide est soumise à une pression de 10 Kbar. Après 24 heures, la cristallisation est totale et le solide obtenu est caractérisé par son point de fusion et par le diagramme de diffraction X sur poudre suivant, mesuré sur un diffractomètre Bruker D5000matic (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°) exp.** | **d (Å) exp.** | **Intensité (%)** |
|---|---|---|
| 5,73 | 15,411 | 11,4 |
| 10,22 | 8,645 | 11,6 |
| 20,10 | 4,413 | 10,2 |
| 23,69 | 3,751 | 59,0 |
| 29,48 | 3,027 | 14,8 |

*Point de fusion : 94°C* Diagramme de spectroscopie infrarouge: 907,5 cm⁻¹ ; 866,7 cm⁻¹ ; 852,8 cm⁻¹ ; 827,4 cm⁻¹ ; 754,6 cm⁻¹ ; 734,6 cm⁻¹ ; 698,4 cm⁻¹ ; 672,1 cm⁻¹ ; 650,9 cm⁻¹ ; 611,9 cm⁻¹ ; 588,1 cm⁻¹.

### Exemple 3 : Composition pharmaceutique

**Formule de préparation pour 1000 comprimés doses à 25 mg :**

| | |
|---|---|
| Composé de l'Exemple 1 ou 2 | 25 g |
| Lactose monohydrate | 62 g |
| Stéarate de Magnésium | 1,3 g |
| Amidon de maïs | 26 g |
| Maltodextrines | 9 g |
| Silice colloïdale anhydre | 0,3 g |
| Amidon de maïs prégélatinisé type A | 4 g |
| Acide stéarique | 2,6 g |

### Exemple 4 : Composition pharmaceutique

**Formule de préparation pour 1000 comprimés doses à 25 mg :**

| | |
|---|---|
| Composé de l'Exemple 1 ou 2 | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

## Revendications

1. Forme cristalline VI de l'agomélatine de formule (I) : 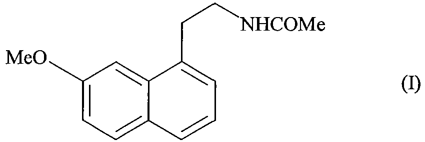 **caractérisée par** le diagramme de diffraction X sur poudre suivant, mesuré sur un diffractomètre Bruker D5000matic (anticathode de cuivre) et exprimé en termes de distance inter-réticulaire d, d'angle de Bragg 2 thêta, et d'intensité relative (exprimée en pourcentage par rapport à la raie la plus intense) :
| **2-Theta (°) exp.** | **d (Å) exp.** | **Intensité (%)** |
|---|---|---|
| 5,73 | 15,411 | 11,4 |
| 10,22 | 8,645 | 11,6 |
| 20,10 | 4,413 | 10,2 |
| 23,69 | 3,751 | 59,0 |
| 29,48 | 3,027 | 14,8 |

2. Procédé de préparation de la forme cristalline VI du composé de formule (I) selon la revendication 1, **caractérisé en ce qu'**une solution d'agomélatine dans l'éther isopropylique est portée à ébullition, refroidie rapidement à 0°C, puis filtrée sous vide.

3. Procédé de préparation de la forme cristalline VI du composé de formule (I) selon la revendication 1, **caractérisé en ce que** l'on cristallise l'agomélatine dans un mélange eau/éthanol (50/50 volume/volume) à température ambiante sous une pression de 10 kbar, pendant 24 heures.

4. Compositions pharmaceutiques contenant comme principe actif la forme cristalline VI de l'agomélatine selon la revendication 1, en combinaison avec un ou plusieurs véhicules inertes, non toxiques et pharmaceutiquement acceptables.

5. Compositions pharmaceutiques selon la revendication 4 utiles pour la fabrication de médicaments pour traiter les troubles du système mélatoninergique.

6. Compositions pharmaceutiques selon la revendication 4 utiles pour la fabrication de médicaments pour le traitement des troubles du sommeil, du stress, de l'anxiété, des dépressions saisonnières ou de la dépression majeure, des pathologies cardiovasculaires, des pathologies du système digestif, des insomnies et fatigues dues aux décalages horaires, de la schizophrénie, des attaques de paniques, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, des troubles de la circulation cérébrale, ainsi que dans les dysfonctionnements sexuels, en tant qu'inhibiteurs de l'ovulation, d'immunomodulateurs et dans le traitement des cancers.

## Patentansprüche

1. Kristallform VI von Agomelatin der Formel (I): 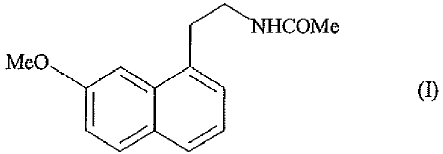 **gekennzeichnet durch** das folgende Pulverröntgenbeugungsdiagramm, gemessen mit einem Bruker-Diffraktometer D5000matic (Kupfer-Antikathode) und ausgedrückt als Netzebenenabstand d, Bragg-Winkel 2 Theta und relative Intensität (ausgedrückt als Prozentsatz bezogen auf die intensivste Bande):
| **2-Theta (°) exp.** | **d (Å) exp.** | **Intensität (%)** |
|---|---|---|
| 5,73 | 15,411 | 11,4 |
| 10,22 | 8,645 | 11,6 |
| 20,10 | 4,413 | 10,2 |
| 23,69 | 3,751 | 59,0 |
| 29,48 | 3,027 | 14,8 |

2. Verfahren zur Herstellung der Kristallform VI der Verbindung der Formel (I) nach Anspruch 1, **dadurch gekennzeichnet, dass** man eine Lösung von Agomelatin in Isopropylether zum Sieden erhitzt, schnell auf 0 °C abkühlt und im Vakuum filtriert.

3. Verfahren zur Herstellung der Kristallform VI der Verbindung der Formel (I) nach Anspruch 1, **dadurch gekennzeichnet, dass** man Agomelatin in einer Wasser/Ethanol-Mischung (50/50 Volumen/Volumen) bei Raumtemperatur unter einem Druck von 10 kbar während 24 Stunden kristallisiert.

4. Pharmazeutische Zubereitungen enthaltend als Wirkstoff die Kristallform VI von Agomelatin nach Anspruch 1 in Kombination mit einem oder mehreren inerten, nichttoxischen und pharmazeutisch annehmbaren Hilfsstoffen.

5. Pharmazeutische Zubereitungen nach Anspruch 4 nützlich für die Herstellung von Arzneimitteln zur Behandlung von Störungen des melatoninergischen Systems.

6. Pharmazeutische Zubereitungen nach Anspruch 4 nützlich für die Herstellung von Arzneimitteln zur Behandlung von Schlafstörungen, Stress, Angst, saisonal bedingten Depressionen oder Major-Depression, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, Schmerzen, psychotischen Störungen, Epilepsie, Diabetes, der Parkinsonschen Krankheit, seniler Demenz, verschiedenen mit dem normalen oder pathologischen Altern verknüpften Störungen, Migräne, Gedächtnisverlusten, der Alzheimerschen Krankheit, Störungen der Gehirndurchblutung sowie sexuellen Dysfunktionen, als Ovulationsinhibitoren, Immunomodulatoren und bei der Behandlung von Krebserkrankungen.

## Claims

1. Crystalline form VI of agomelatine of formula (I): 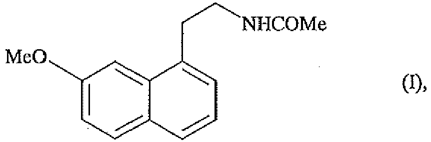 **characterised by** the following X-ray powder diffraction diagram, measured using a Bruker D5000matic diffractometer (copper anticathode) and expressed in terms of interplanar distance d, Bragg's angle 2 theta and relative intensity (expressed as a percentage relative to the most intense line):
| **2**-**Theta (°) exp.** | **d (Å) exp.** | **Intensity (%)** |
|---|---|---|
| 5.73 | 15.411 | 11.4 |
| 10.22 | 8.645 | 11.6 |
| 20.10 | 4.413 | 10.2 |
| 23.69 | 3.751 | 59.0 |
| 29.48 | 3.027 | 14.8 |

2. Process for the preparation of the crystalline form VI of the compound of formula (I) according to claim 1, **characterised in that** a solution of agomelatine in isopropyl ether is heated at boiling, rapidly cooled to 0°C and then filtered *in vacuo.*

3. Process for the preparation of the crystalline form VI of the compound of formula (I) according to claim 1, **characterised in that** agomelatine is crystallised from a water/ethanol mixture (50/50 volume/volume) at ambient temperature under a pressure of 10 kbar for 24 hours.

4. Pharmaceutical compositions comprising as active ingredient the crystalline form VI of agomelatine according to claim 1, in combination with one or more pharmaceutically acceptable, inert and non-toxic carriers.

5. Pharmaceutical compositions according to claim 4 for use in the manufacture of medicaments for treating disorders of the melatoninergic system.

6. Pharmaceutical compositions according to claim 4 for use in the manufacture of medicaments for the treatment of sleep disorders, stress, anxiety, seasonal affective disorder or major depression, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, cerebral circulation disorders or sexual dysfunctions, as ovulation-inhibitors or immunomodulators, or in the treatment of cancers.
